(19) Europäisches Patentamt
European Patent Office
Office européen des brevets

(11) EP 4 434 989 A1

(12) EUROPEAN PATENT APPLICATION
published in accordance with Art. 153(4) EPC

(43) Date of publication:
**25.09.2024 Bulletin 2024/39**

(21) Application number: **22910008.6**

(22) Date of filing: **20.12.2022**

(51) International Patent Classification (IPC):
*C07D 487/04 (2006.01)* *C07D 471/20 (2006.01)*
*C07D 471/04 (2006.01)* *A61K 31/5025 (2006.01)*
*A61P 35/00 (2006.01)*

(52) Cooperative Patent Classification (CPC):
**A61K 31/5025; A61P 35/00; C07D 471/04; C07D 471/20; C07D 487/04**

(86) International application number:
**PCT/CN2022/140380**

(87) International publication number:
**WO 2023/116696 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.12.2021 CN 202111571838**
**21.12.2021 CN 202111571406**

(71) Applicant: **Nanjing Chia Tai Tianqing Pharmaceutical Co., Ltd.**
**Jiangsu 210046 (CN)**

(72) Inventors:
- **MA, Changyou**
  **Nanjing, Jiangsu 210046 (CN)**
- **HUANG, Dandan**
  **Nanjing, Jiangsu 210046 (CN)**
- **WU, Yebin**
  **Nanjing, Jiangsu 210046 (CN)**
- **SU, Jincai**
  **Nanjing, Jiangsu 210046 (CN)**
- **MA, Li**
  **Nanjing, Jiangsu 210046 (CN)**
- **DAI, Qingyu**
  **Nanjing, Jiangsu 210046 (CN)**
- **ZUO, Rui**
  **Nanjing, Jiangsu 210046 (CN)**
- **PEI, Junjie**
  **Nanjing, Jiangsu 210046 (CN)**
- **MIAO, Lei**
  **Nanjing, Jiangsu 210046 (CN)**
- **WU, Jian**
  **Nanjing, Jiangsu 210046 (CN)**
- **XU, Dan**
  **Nanjing, Jiangsu 210046 (CN)**
- **ZHU, Chunxia**
  **Nanjing, Jiangsu 210046 (CN)**
- **TIAN, Zhoushan**
  **Nanjing, Jiangsu 210046 (CN)**

(74) Representative: **Elzaburu S.L.P.**
**Paseo de la Castellana 259C**
**Torre de Cristal, planta 28**
**28046 Madrid (ES)**

# (54) METHIONINE ADENOSYLTRANSFERASE 2A HETEROCYCLIC INHIBITOR

(57) Disclosed is a methionine adenosyltransferase 2A heterocyclic inhibitor. The structure of the methionine adenosyltransferase 2A inhibitor is represented by general formula I, wherein at most two of W, X, Y and Z are simultaneously N, and $R^1$ and $R^2$ are each independently selected from a 6- to 10-membered aryl or a 9- to 18-membered benzoheterocyclyl. Also disclosed is a preparation method therefor. The compound of general formula I has significant inhibitory activity of methionine adenosyltransferase 2A and can be used for treating methionine adenosyltransferase 2A-mediated diseases.

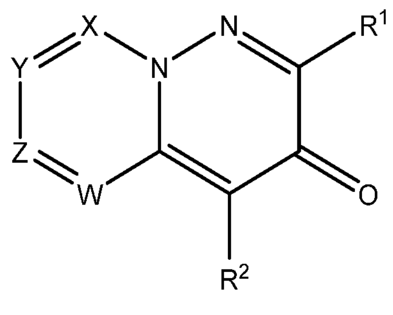

I

EP 4 434 989 A1

## Description

### Technical Field

The present disclosure belongs to the field of medical technology, and specifically relates to heterocyclic inhibitors of methionine adenosyltransferase 2A, a preparation method and use thereof.

### Background

Methionine adenosyltransferase (MAT) (also known as S-adenosylmethionine synthase) is a cellular enzyme that catalyzes the synthesis of S-adenosylmethionine (SAM or AdoMet) from methionine and ATP and is considered the rate-limiting factor in the methionine cycle step. SAM is the propylamino donor in polyamine biosynthesis and is the major methyl donor for DNA methylation, and it is involved in gene transcription and cell proliferation as well as the production of secondary metabolites. The three human isoenzymes of MAT include MAT1, MAT2, and MAT3, of which MAT1 and MAT3 are expressed in liver tissue. Methionine adenosyltransferase 2A (MAT2A), an isoform of MAT2, is ubiquitously expressed in various types of human cells and it is the predominant form of human cancer and plays a key role in the generation of SAM.

MTAP (methylthioadenosine phosphorylase) is an enzyme widely expressed in normal tissues. It catalyzes the conversion of methylthioadenosine (MTA) into adenine and 5-methylthioribose-1-phosphate, adenine into adenosine monophosphate, and 5-methylthioribose-1-phosphate into methionine and formate. MTA can be used as an alternative purine source when purine synthesis is blocked, for example by antimetabolites. The gene encoding MTAP is located in a site on chromosome 9 that is frequently deleted in cancer patients from cells in the central nervous system, pancreas, esophagus, bladder, and lungs. Loss of MTAP leads to the accumulation of MTA compared with MTAP-expressing cells, making MTAP-deficient cells more dependent on SAM production and therefore more dependent on MAT2A activity. In a screen of approximately 400 cancer cell lines, MAT2A knockdown resulted in a greater percentage loss of viability in MTAP-deficient cells compared with cells that normally expressed MTAP. Furthermore, inducible knockdown of MAT2A protein reduced tumor growth in vivo. These results suggest that MAT2A inhibitors may provide a novel treatment approach for patients with MTAP-deficient tumors. Currently, Chinese Patent Application CN109890822A discloses a pyrazolopyrimidinone MAT2A inhibitor, and WO2020123395A1 discloses a 2-oxoquinazoline derivative as a MAT2A inhibitor. The present disclosure provides a new heterocyclic methionine adenosyltransferase 2A inhibitor.

### SUMMARY OF THE INVENTION

The present disclosure provides a compound of formula I or a pharmaceutically acceptable salt thereof:

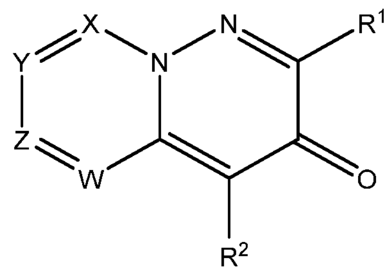

I

wherein X is selected from $CR^3$ or N; Y is selected from $CR^4$ or N; Z is selected from $CR^5$ or N; W is selected from $CR^6$ or N;

wherein $R^3$, $R^4$, $R^5$ and $R^6$ are independently selected from hydrogen, cyano, C2-C6 alkynyl, halogen, hydroxyl, $-NH_2$, (C1-C6 alkyl)-$NR^7$-, (C1-C6 alkyl)-O-, (C1-C6 alkyl)-S-, C1-C6 alkyl, C3-C6 cycloalkyl, 6- to 10-membered aryl, C2-C6 alkenyl or C3-C6 cycloalkenyl; the C1-C6 alkyl, C2-C6 alkenyl, C3-C6 cycloalkyl or C3-C6 cycloalkenyl itself or as part of another group is optionally substituted by halogen, cyano, hydroxyl, $-NR^7R^8$, C1-C3 alkyl, C1-C3 alkoxy, C2-C6 alkenyl or C2-C6 alkynyl, the 6- to 10-membered aryl is optionally substituted by halogen, hydroxyl, cyano, $-NR^7R^8$, $-NO_2$, C1-C3 alkyl, C1-C3 alkoxy, C2-C6 alkenyl or C2-C6 alkynyl, wherein the C1-C3 alkyl, C1-C3 alkoxy, C2-C6 alkenyl or C2-C6 alkynyl is optionally substituted by halogen, hydroxyl, cyano, $-NR^7R^8$ or $-NO_2$;

$R^1$ and $R^2$ are independently selected from 6- to 10-membered aryl or 9- to 18-membered benzoheterocyclyl, the 6- to 10-membered aryl or 9- to 18-membered benzoheterocyclyl is optionally substituted by halogen, hydroxyl, cyano, $-NR^7R^8$, $-NO_2$, $-NR^9C(O)R^{10}$, C1-C6 alkyl, (C1-C6 alkyl)-O-, $-C(O)NR^9R^{10}$ or 5- to 7-membered heteroaryl,

the C1-C6 alkyl itself or as part of another group or the 5- to 7-membered heteroaryl is optionally substituted by halogen, cyano, hydroxyl, C1-C3 alkyl, (C1-C3 alkyl) -O- or $-NR^7R^8$;
$R^7$, $R^8$, $R^9$ and $R^{10}$ are independently selected from H or C1-C6 alkyl;
provided that: at most 2 of W, X, Y and Z are simultaneously N.

In some embodiments, X is selected from $CR^3$.
In some embodiments, Y is selected from $CR^4$.
In some embodiments, Z is selected from $CR^5$.
In some embodiments, W is N.
In some embodiments, $R^3$, $R^4$, $R^5$ and $R^6$ are independently selected from hydrogen, halogen, hydroxyl, $-NH_2$, (C1-C6 alkyl)-$NR^7$-, (C1-C6 alkyl)-O-, C1-C6 alkyl, C3-C6 cycloalkyl or 6- to 10-membered aryl, wherein the C1-C6 alkyl itself or as part of another group or the C3-C6 cycloalkyl is optionally substituted by halogen, cyano, hydroxyl or $-NR^7R^8$, the 6-to 10-membered aryl is optionally substituted by halogen-substituted C1-C3 alkoxy.
In some typical embodiments, $R^3$, $R^4$, $R^5$ and $R^6$ are independently selected from hydrogen, halogen, hydroxyl, $-NH_2$, (C1-C6 alkyl)-$NR^7$-, (C1-C6 alkyl)-O-, C1-C6 alkyl, C3-C6 cycloalkyl or 6- to 10-membered aryl, wherein the C1-C6 alkyl itself or as part of another group or the C3-C6 cycloalkyl is optionally substituted by halogen, the 6- to 10-membered aryl is optionally substituted by halogen-substituted C1-C3 alkoxy.
In some typical embodiments, $R^3$, $R^4$, $R^5$ and $R^6$ are independently selected from hydrogen, halogen, hydroxyl, $-NH_2$, (C1-C6 alkyl)-$NR^7$-, (C1-C6 alkyl)-O-, C1-C6 alkyl, C3-C6 cycloalkyl or 6- to 10-membered aryl, wherein the C1-C6 alkyl itself or as part of another group or the C3-C6 cycloalkyl is optionally substituted by fluorine, the 6- to 10-membered aryl is optionally substituted by fluorine-substituted C1-C3 alkoxy.
In some more typical embodiments, $R^3$ is selected from hydrogen, C1-C6 alkyl or C3-C6 cycloalkyl; preferably, $R^3$ is selected from hydrogen or C1-C6 alkyl.
In some more typical embodiments, $R^3$ is selected from hydrogen, methyl or cyclopropyl; preferably, $R^3$ is selected from hydrogen or methyl; more preferably, $R^3$ is hydrogen.
In some more typical embodiments, $R^4$ is selected from hydrogen or C1-C6 alkyl; preferably, $R^4$ is selected from hydrogen or methyl; more preferably, $R^4$ is hydrogen.
In some more typical embodiments, $R^5$ is selected from hydrogen, halogen, hydroxyl, (C1-C6 alkyl)-$NR^7$-, (C1-C6 alkyl)-O-, C3-C6 cycloalkyl or 6- to 10-membered aryl, wherein the C1-C6 alkyl itself or as part of another group or the C3-C6 cycloalkyl is optionally substituted by fluorine, the 6- to 10-membered aryl is optionally substituted by fluorine-substituted C1-C3 alkoxy.
In some more typical embodiments, $R^5$ is selected from hydrogen, chlorine, hydroxyl, cyclopropyl, $CF_3CH_2O$-, $CHF_2O$-, $CF_3CH_2NH$-, 4-difluoromethoxyphenyl or $CH_3CH_2O$-; preferably, $R^5$ is selected from cyclopropyl, $CF_3CH_2O$-, $CF_3CH_2NH$- or $CH_3CH_2O$-; more preferably, $R^5$ is selected from cyclopropyl, $CF_3CH_2O$- or $CF_3CH_2NH$-; most preferably, $R^5$ is cyclopropyl.
In some embodiments, $R^1$ and $R^2$ are independently selected from phenyl,

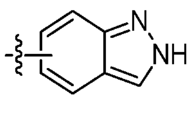

or

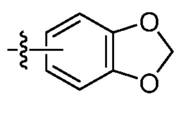

,

wherein the groups are optionally substituted by halogen, hydroxyl, cyano, $-NR^7R^8$, $-NO_2$, $-NR^9C(O)R^{10}$, C1-C6 alkyl, (C1-C6 alkyl)-O-, $-C(O)NR^9R^{10}$ or 5- to 7-membered heteroaryl, the C1-C6 alkyl itself or as part of another group or the 5- to 7-membered heteroaryl is optionally substituted by halogen, cyano, hydroxyl, C1-C3 alkyl, (C1-C3 alkyl) -O- or $-NR^7R^8$.
In some embodiments, $R^1$ and $R^2$ are independently selected from phenyl,

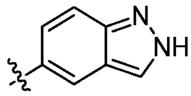,

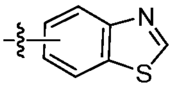, 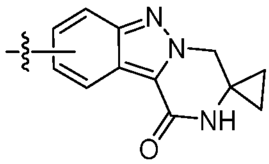, 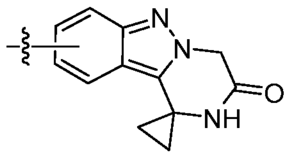, 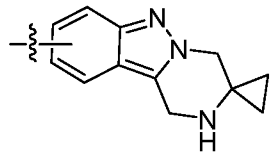,

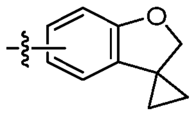 or 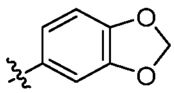

group, wherein the groups are optionally substituted by C1-C6 alkyl or (C1-C6 alkyl)-O-, the C1-C6 alkyl itself or as part of another group is optionally substituted by halogen.

In some embodiments, $R^7$, $R^8$, $R^9$ and $R^{10}$ are independently selected from H.

In some embodiments, $R^1$ is selected from phenyl,

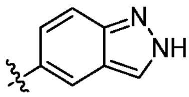 or 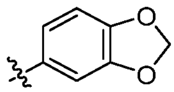

group, wherein the groups are optionally substituted by C1-C6 alkyl or (C1-C6 alkyl)-O-, the C1-C6 alkyl itself or as part of another group is optionally substituted by halogen.

In some embodiments, $R^1$ is selected from

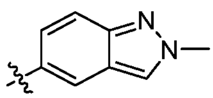, 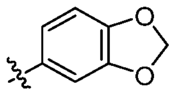

or

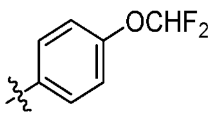;

preferably, $R^1$ is

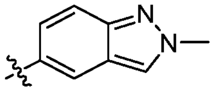.

In some embodiments, $R^2$ is selected from phenyl or

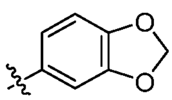

group, wherein the groups are optionally substituted by (C1-C6 alkyl)-O-, the C1-C6 alkyl is optionally substituted by halogen.

In some embodiments, $R^2$ is selected from

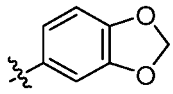 or 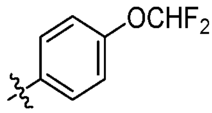 ;

preferably, $R^2$ is

; 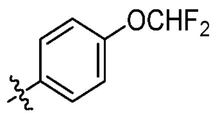 .

In some embodiments, the foregoing compound of formula I having the structure of formula II,

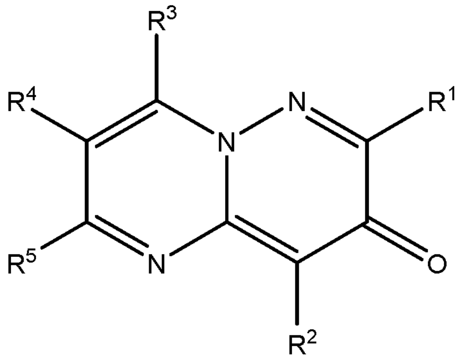

II

wherein $R^1$, $R^2$, $R^3$, $R^4$ and $R^5$ are defined as the compound of formula I.

On the other hand, the present disclosure provides a compound selected from the following group, or a pharmaceutically acceptable salts thereof:

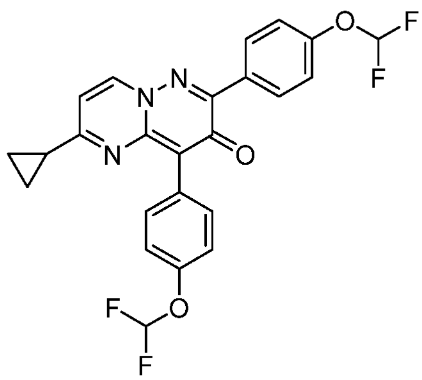 , 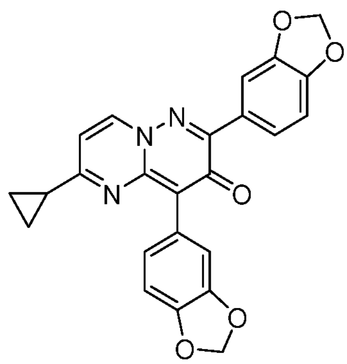

and

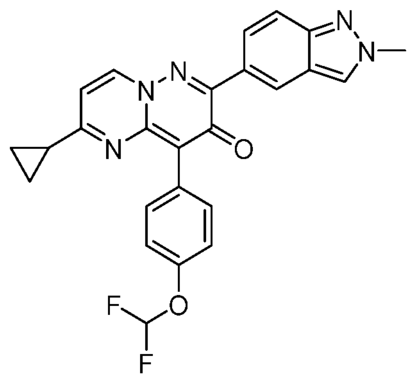

.

In some embodiments, the present disclosure provides a pharmaceutical composition, comprising a therapeutically effective amount of the above compound or a pharmaceutically acceptable salt thereof.

In some embodiments, the present disclosure provides a pharmaceutical composition, comprising a therapeutically effective amount of the above compound or a pharmaceutically acceptable salt thereof and a pharmaceutically acceptable carrier.

The pharmaceutical composition in the present disclosure can be administered by any suitable route or method, such as by oral or parenteral (e.g., intravenous) administration. The therapeutically effective amount of the compound above is from about 1 mg to 1 g/kg body weight/day.

For oral administration, the pharmaceutical composition in the present disclosure is typically provided in the form of tablets, capsules or solutions. Tablets may contain the compound of the present disclosure or a pharmaceutically acceptable salt thereof and a pharmaceutically acceptable carrier. The carrier includes, but is not limited to, diluents, disintegrants, binders, lubricants.

For parenteral administration, the pharmaceutical composition in the present disclosure can be administered by intravenous injection, intramuscular injection or subcutaneous injection. The pharmaceutical composition is usually provided as a sterile aqueous solution, suspension or lyophilized powder, with appropriate pH and isotonicity adjusted.

On the other hand, the present disclosure also provides the use of the above compound, a pharmaceutically acceptable salt thereof or the pharmaceutical composition in the preparation of a medicament for preventing and/or treating a MAT2A-mediated disease or condition.

On the other hand, the present disclosure also provides a method for preventing and/or treating a MAT2A-mediated disease or condition, comprising administering to a subject in need an effective amount of the above compound, a pharmaceutically acceptable salt thereof or the pharmaceutical composition.

On the other hand, the present disclosure also provides the above compound, a pharmaceutically acceptable salt thereof or the pharmaceutical composition in the present disclosure for use in the prevention and/or treatment of a MAT2A-mediated disease or condition. Examples of the MAT2A-mediated disease or condition include colorectal cancer, etc.

In some specific embodiments, the MAT2A-mediated disease is a disease mediated by overexpression of MAT2A.

On the other hand, the present disclosure provides a method for preparing compounds of formula I or II, including but not limited to the following synthesis scheme:

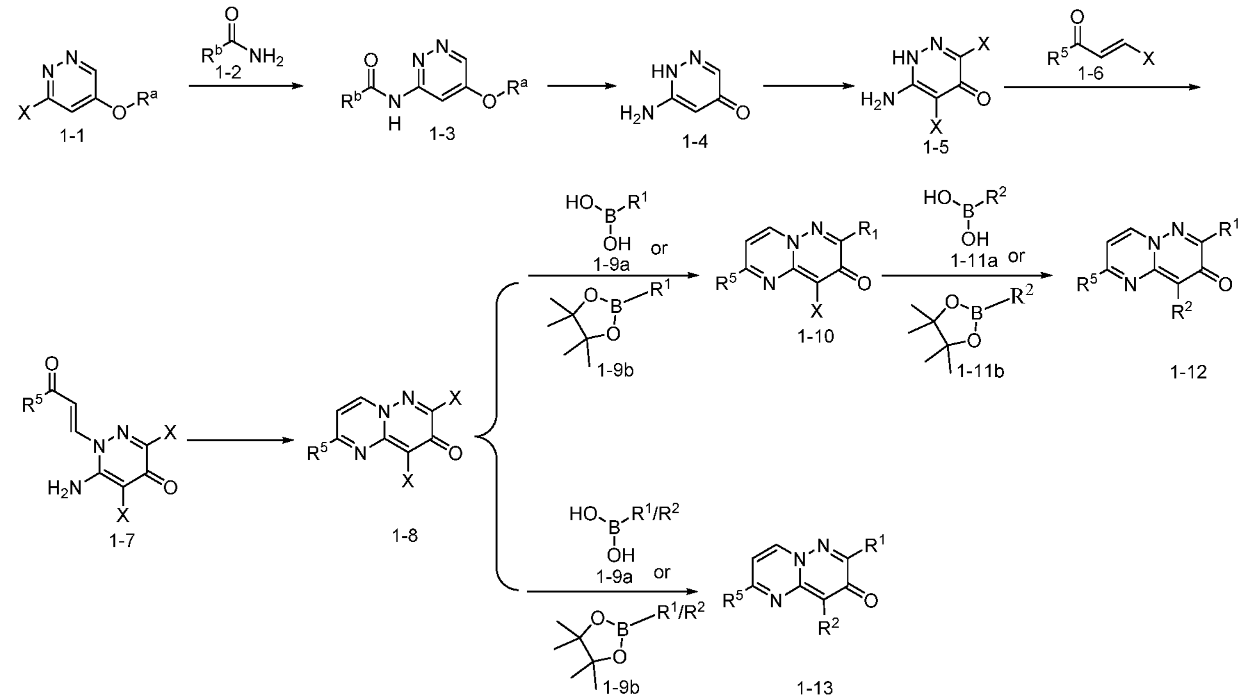

1-1 1-3 1-4 1-5 1-7 1-8 1-10 1-12 1-13

wherein X is selected from chlorine, bromine or iodine, $R^1$, $R^2$ and $R^5$ are defined as in formula I above, $R^a$ and $R^b$ are independently selected from C1-C3 alkyl.

In some embodiments, $R^a$ and $R^b$ are independently selected from methyl.

The compound of formula 1-1 reacts with the compound of formula 1-2 to generate the compound of formula 1-3, and the compound of formula 1-3 generates the compound of formula 1-4 with hydrohalic acid; the compound of formula 1-5 was obtained through halogenation reaction of compound of formula 1-4; the compound of formula 1-5 reacts with the compound of formula 1-6 to obtain the compound of formula 1-7; the compound of formula 1-8 was obtained through cyclization reaction of formula 1-7; the compound of formula 1-8 reacts with the compound of formula 1-9a or formula 1-9b to obtain the compound of formula 1-10; the compound of formula 1-10 reacts with the compound of formula 1-lla or formula 1-llb obtain the compound of formula 1-12; or

the compound of formula 1-8 reacts with the compound of formula 1-9a or formula 1-9b to obtain the compound of formula 1-13, wherein in the compound of formula 1-13, $R^1$ and $R^2$ are the same.

In some embodiments, X is chlorine.

In some embodiments, $R^1$ and $R^2$ are independently selected from

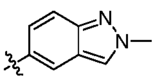,
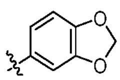

or

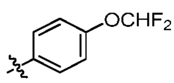,

$R^5$ is selected from cyclopropyl.

**Brife Description of the Drawings**

Figure 1 is the tumor inhibition curve of HCT116 $MTAP^{-/-}$ transplanted tumor model in Test Example 3.
Figure 2 is the intratumorally SAM inhibition result of HCT116 $MTAP^{-/-}$ transplanted tumor model in Test Example 3.
Figure 3 is the tumor inhibition curve of KP-4 transplanted tumor model in Test Example 4.

Figure 4 is the intratumorally SAM inhibition result of KP-4 transplanted tumor model in Test Example 4.

**Related definitions**

Unless otherwise specified, the following terms used in the description and claims have the following meanings: The term "optionally" or "optionally (substituted by)" means that the subsequently described event or circumstance may or may not occur, and that the description includes both the occurrence and the absence of the stated event or circumstance.

Numerical ranges in the present disclosure refer to each integer within the given range. For example, "C1-C6" means that the group having 1 carbon atom, 2 carbon atoms, 3 carbon atoms, 4 carbon atoms, 5 carbon atoms or 6 carbon atoms; "C3-C6" means that the group having 3 carbon atoms, 4 carbon atoms, 5 carbon atoms, or 6 carbon atoms.

The term "membered" refers to the number of skeletal atom or atomic group that make up the ring. For example, "5- to 7-membered" means that the number of skeletal atom or atomic that make up the ring is 5, 6, or 7. Therefore, for example, pyridine, piperidine, piperazine and benzene are six-membered rings, while thiophene and pyrrole are five-membered rings.

The term "substituted" means that any one or more hydrogen atoms on a specific group are substitued by a substituent, as long as the valence state of the specific group is normal, and the substituted compound is stable. For example, "substituted by halogen" means that any one or more hydrogen atoms on a specific group are substitued by halogen, as long as the valence state of the specific group is normal, and the substituted compound is stable.

The term "alkyl" refers to a saturated aliphatic hydrocarbon group, including straight-chain or branched saturated hydrocarbon groups, having the number of carbon atoms as shown. For example, the term "C1-C6 alkyl" includes C1 alkyl, C2 alkyl, C3 alkyl, C4 alkyl, C5 alkyl or C6 alkyl, examples include, but are not limited to, methyl, ethyl, n-propyl, isopropyl, n-butyl, isobutyl, tert-butyl, n-pentyl, 2-pentyl, 3-pentyl, n-hexyl, 2-hexyl or 3-hexyl, etc. The term "cycloalkyl" refers to a monocyclic saturated hydrocarbon system, no heteroatoms, and no double bonds. Examples of the term "3- to 6-membered cycloalkyl" include, but are not limited to, cyclopropyl, cyclobutyl, cyclopentyl or cyclohexyl. The term "halogen" refers to fluorine, chlorine, bromine and iodine.

The term "aryl" refers to an all-carbon monocyclic or fused bicyclic aromatic ring group having a conjugated π electron system, which is obtained by removing a hydrogen atom from a single carbon atom of the parent aromatic ring system. This term includes bicyclic groups fused with saturated rings, partially unsaturated rings or aromatic carbocyclic rings; examples include, but are not limited to, phenyl, naphthyl, anthracenyl, indanyl, indane, 1,2-dihydronaphthalene or 1,2,3,4-tetralin.

The term "heteroaryl" refers to a monovalent aryl group containing at least one heteroatom independently selected from nitrogen, oxygen and sulfur. For example, examples of "5- to 7-membered heteroaryl" include, but are not limited to, pyridyl, thienyl, imidazolyl, pyrimidinyl, pyridyl, furyl, pyrazinyl or thiazolyl.

The term "9- to 18-membered benzoheterocyclyl" refers to a ring system with 9 to 18 ring atoms or ring atomic groups formed by the fusion of a benzene ring and a heterocyclic ring. The benzene ring and the heterocyclic ring share a pair of adjacent ring atoms. And the connection point of the benzoheterocyclyl with the parent core structure is in the benzene ring part. Wherein the heterocyclic part is a 5- to 12-membered saturated, partially unsaturated or completely unsaturated ring system having ring carbon atoms and 1 to 4 ring heteroatoms or heteroatomic groups. The heteroatoms or heteroatomic groups are independently selected from nitrogen, sulfur, oxygen, sulfoxide, sulfone,

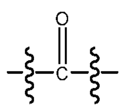,

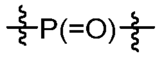 or 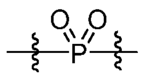.

The heterocyclic ring can be monocyclic, bicyclic or tricyclic systems, wherein a cyclic system having two or more rings exist in the form of fused ring, spiro ring or bridged ring. Examples include, but are not limited to,

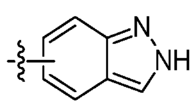

or

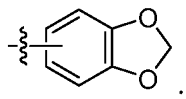

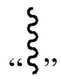

in "

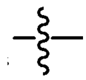

" refers to the chemical bond connection point. When

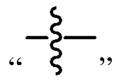

appears in a bicyclic or polycyclic ring and the connection point is uncertain, it means that the connection site is limited to any atom on the single ring where

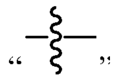

' is located, as long as the valence of the atom allows. For example,

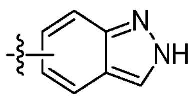

indicates that the connection point is only located at any carbon atom on the benzene ring of the bicyclic ring and must meet the requirements of atomic valence bonds.

The term "pharmaceutically acceptable salts" refers to salts that retain the biological potency of the free acids and bases of a particular compound without adverse biological effects. For example, acid (including organic and inorganic acids) addition salts or base addition salts (including organic and inorganic bases).

The pharmaceutically acceptable salts of the present disclosure can be obtained by conventional chemical methods from parent compound containing acid group or basic group. In general, such salts are prepared by reacting the free acid or free base of these compounds with a stoichiometric amount of the appropriate base or acid in water or an organic solvent or a mixture of the two.

The term "effective amount" or "therapeutically effective amount" refers to a nontoxic amount of a drug or an agent that is effective in achieving the desired effect.

The term "pharmaceutically acceptable carrier" refers to those carriers that have no obvious irritating effect on the body and do not impair the biological activity and performance of the active compound. Including but not limited to any diluent, disintegrant, binder, glidant, and wetting agent approved by the National Medical Products Administration for use in humans or animals.

The abbreviations used in the claims and description have the following meanings:

M: mol/L;
mM: mmol/L;
μM: μmol/L;
nM: nmol/L;

LCMS: Liquid Chromatography-Mass Spectrometry;
Brij35: polyoxyethylene lauryl ether;
BSA: Bovine Serum Albumin;
DMSO: dimethyl sulfoxide;
rpm: revolutions/minute;
Tris-HCl: Tris-(hydroxymethyl)-aminomethan-hydrochlorid;
$OD_{620}$: absorbance value at 620 nm wavelength;
SAM: S-Adenosyl methionine or S-Adenosylmethionine.

## Detailed Description

The preparation methods of the compounds of the present disclosure are described in more details below, but these specific preparation methods do not constitute any limitation on the scope of the present disclosure. In addition, reaction conditions such as reactants, solvents, bases, amounts of compounds used, reaction temperature, reaction time, etc. are not limited to the following examples.

The compounds of the present disclosure can also be prepared by optionally combining various synthetic methods described in the present disclosure or known in the art, and such combinations can be easily performed by those skilled in the art.

### Example 1: 2-cyclopropyl-7,9-bis(4-(difluoromethoxy)phenyl)-8H-pyrimido[1,2-b]pyridazin-8-one

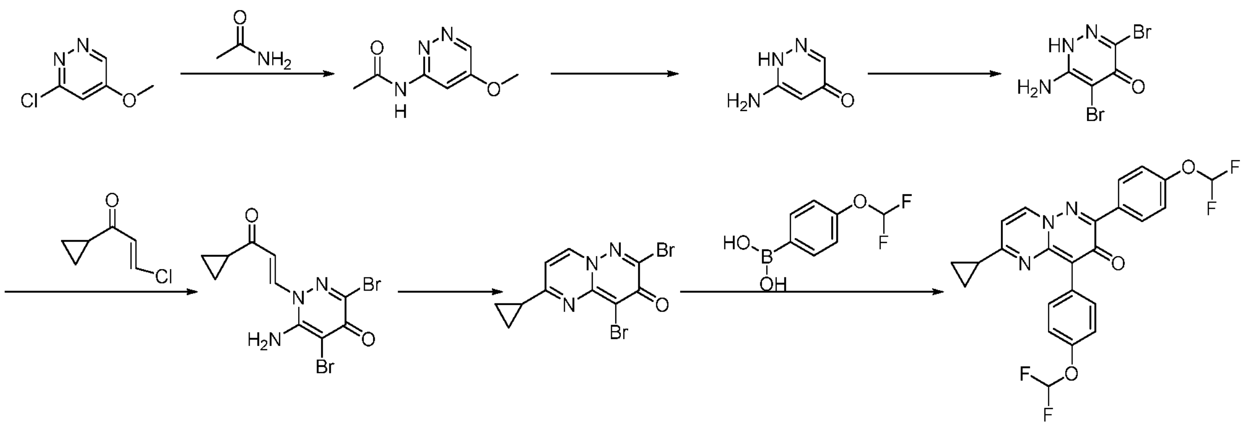

a) a) Preparation of N-(5-methoxypyridazin-3-yl)acetamide 3-Chloro-5-methoxypyridazine (1g), acetamide (0.61g), Tris(dibenzylideneacetone)dipalladium (0.32g), 4,5-bisdiphenylphosphine-9,9-dimethylxanthene (0.40g) and cesium carbonate (6.76g) were added to the reaction flask successively, then with 1,4-dioxane (100mL) added, under the protection of nitrogen, the reaction mixture was stirred at 100°C for 3 h. The evaporated residue was obtained through vacuum concentration, extracted with ethyl acetate (3×50mL). The organic phases were combined, washed with saturated brine (1 × 50mL), dried over anhydrous sodium sulfate and filtered. The filtrate was concentrated through vacuum concentration and purified by silica gel column chromatography (mobile phase: ethyl acetate/petroleum ether =10/1 (V/V)) to obtain the title compound (780mg).
LCMS m/z= 168.05$[M+1]^+$
b) Preparation of 6-aminopyridazin-4(1H)-one N-(5-Methoxypyridazin-3-yl)acetamide (500mg) and hydrogen bromide aqueous solution (15mL) were added to microwave tube. The reaction mixture was stirred at 140°C for 5 h under microwave irradiation, concentrated through vacuum concentration to obtain the title compound (360mg).
LCMS m/z= 111.95$[M+1]^+$
c) Preparation of 6-amino-3,5-dibromopyridazin-4(1H)-one
To a solution of 6-aminopyridazin-4(1H)-one (360mg) and N-bromosuccinimide (1.73g) in N,N-dimethylformamide (4mL) were added to the reaction flask. The reaction mixture was stirred at room temperature for 2 h, concentrated and dired through vacuum concentration to obtain residue. The residue was washed with acetonitrile (3×5mL) to obtain the title compound (360mg).
d) Preparation of 6-amino-3,5-dibromo-1-((1E)-3-cyclopropyl-3-oxoprop-1-en-1-yl)pyridazin-4(1H)-one
To a solution of 6-amino-3,5-dibromopyridazin-4(1H)-one (430mg), (2E)-3-chloro-1-cyclopropylprop-2-en-1-one

(167.0mg) and potassium carbonate (663.0mg) in N,N-dimethylformamide (43mL) were added to the reaction flask. The reaction mixture was stirred at 25°C overnight, concentrated through vacuum concentration to obtain a mixture. The mixture was adjusted to pH = 2~3 with 1M hydrochloric acid solution and washed with water (3×10mL) to obtain the title compound (335mg).

e) Preparation of 7,9-dibromo-2-cyclopropyl-8H-pyrimido[1,2-b]pyridazin-8-one 6-Amino-3,5-dibromo-1-((1E)-3-cyclopropyl-3-oxoprop-1-en-1-yl)pyridazin-4(1H)-one (355mg) was added to the reaction flask, then with a 2mol/L solution of hydrogen chloride in 1,4-dioxane (10mL) added, the reaction mixture was stirred at 25°C for 1 h, concentrated through vacuum concentration. The evaporated residue was washed with sodium bicarbonate aqueous solution (1×10mL) and filtered. The filter cake was collected and washed with water (3×5mL) to obtain the title compound (320mg).

f) Preparation of 2-cyclopropyl-7,9-bis(4-(difluoromethoxy)phenyl)-8H-pyrimido[1,2-b]pyridazin-8-one 7,9-Dibromo-2-cyclopropyl-8H-pyrimido[1,2-b]pyridazin-8-one (60mg), (4-(difluoromethoxy)phenyl)boronic acid (98.1mg), water (0.4mL), 1,4-dioxane (2mL), potassium phosphate (184.6mg) and [1,1'-bis(diphenylphosphine)ferrocene]paladium dichloride dichloromethane complex (14.17mg) were added to the reaction flask. Under the protection of nitrogen, the reaction mixture was stirred at 80°C for 1 h and concentrated through vacuum concentration to obtain evaporated residue. The residue was purified through preparative separation to obtain the title compound (37mg). Preparative separation conditions: column: XBridge PrepOBD C18, 30*150mm, 5μm; column temperature: 25°C; mobile phase A: water (10mmol/L, $NH_4HCO_3$), mobile phase B: acetonitrile; flow rate: 60mL/min; the elution gradient is 45%B~85%B from 0 to 10 min, and 85%B after 10 min; detection wavelength: UV220nm; retention time (min): 7.32.

$^1$H NMR (400 MHz, DMSO-$d_6$) δ 8.72 (d, $J$ = 7.2 Hz, 1H), 8.27 - 8.21 (m, 2H), 7.64 - 7.58 (m, 2H), 7.56 - 7.26 (m, 3H), 7.21 - 7.11 (m, 3H), 7.07 (d, $J$ = 7.2 Hz, 1H), 2.20 (tt, $J$ = 8.1, 4.5 Hz, 1H), 1.18 - 1.10 (m, 2H), 1.01 (p, $J$ = 3.8 Hz, 2H). LCMS m/z= 472 $[M+H]^+$

**Example 2: 7,9-bis(benzo[d][1,3]dioxol-5-yl)-2-cyclopropyl-8H-pyrimido[1,2-b]pyridazin-8-one**

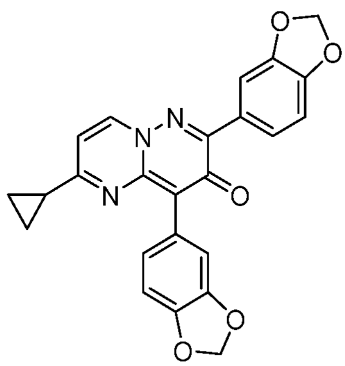

According to the preparation method of Example 1, replacing 4-(difluoromethoxy)phenylboronic acid in step f) with benzo[d][1,3]dioxol-5-ylboronic acid

( 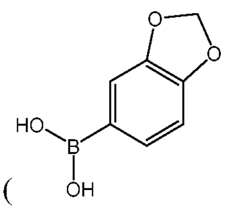 )

to obtain the title compound.

$^1$H NMR (400 MHz, DMSO-$d_6$) δ 8.69 (d, $J$ = 7.2 Hz, 1H), 7.88 (dd, $J$ = 8.3, 1.7 Hz, 1H), 7.76 (d, $J$ = 1.7 Hz, 1H), 7.09 - 6.99 (m, 4H), 6.93 (d, $J$ = 8.0 Hz, 1H), 6.11 (s, 2H), 6.04 (s, 2H), 2.18 (td, $J$ = 8.0, 4.1 Hz, 1H), 1.12 (dt, $J$ = 6.7, 3.4 Hz, 2H), 1.01 (t, $J$ = 3.8 Hz, 2H). LCMS m/z= 428 $[M+1]^+$

**Example 3: 2-cyclopropyl-9-(4-(difluoromethoxy)phenyl)-7-(2-methyl-2H-indazol-5-yl)-8H-pyrimido[1,2-b]pyridazin-8-one**

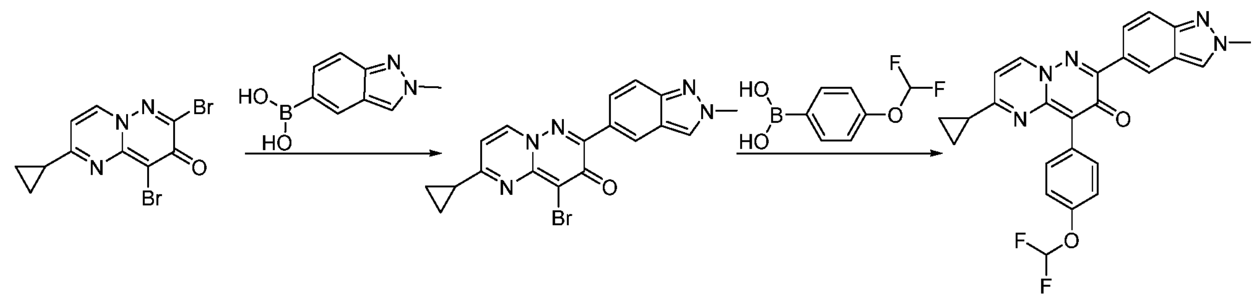

a) Preparation of 9-bromo-2-cyclopropyl-7-(2-methyl-2H-indazol-5-yl)-8H-pyrimido[1,2-b]pyridazin-8-one 7,9-Dibromo-2-cyclopropyl-8H-pyrimido[1,2-b]pyridazin-8-one (50mg), (2-methyl-2H-indazol-5-yl)boronic acid (28.06mg), potassium carbonate (60.09mg), [1,1'-bis(diphenylphosphine)ferrocene]palladium dichloride dichloromethane complex (11.81mg), 1,4-dioxane (0.5mL) and water (0.1mL) were added to the reaction flask successively. Under the protection of nitrogen, the reaction mixture was stirred at 60°C for 1 h, concentrated through vacuum concentration and purified by silica gel column chromatography (mobile phase: ethyl acetate/petroleum ether =4/1 (V/V) ) to obtain the title compound (40mg).

$^1$H NMR (400 MHz, DMSO) δ 8.98 (s, 1H), 8.81 (d, J = 7.1 Hz, 1H), 8.54 (s, 1H), 7.95 (d, J = 8.9 Hz, 1H), 7.66 (d, J = 9.1 Hz, 1H), 7.14 (d, J = 7.1 Hz, 1H), 4.20 (s, 3H), 2.34 (s, 1H), 1.46 - 1.10 (m, 4H).
LCMS m/z= 396[M+1]$^+$

b) Preparation of 2-cyclopropyl-9-(4-(difluoromethoxy)phenyl)-7-(2-methyl-2H-indazol-5-yl)-8H-pyrimido[1,2-b]pyridazin-8-one 9-Bromo-2-cyclopropyl-7-(2-methyl-2H-indazol-5-yl)-8H-pyrimido[1,2-b]pyridazin-8-one (35mg), (4-(difluoromethoxy)phenyl)boronic acid (24.90mg), potassium carbonate (36.62mg), [1,1'-bis(diphenylphosphine)ferrocene]palladium dichloride dichloromethane complex (7.20mg), 1,4-dioxane (1mL) and water (0.2mL) were added to the reaction flask successively. Under the protection of nitrogen, the reaction mixture was stirred at 80°C for 1 h, concentrated through vacuum concentration and purified through preparative separation to obtain the title compound (14.4mg). Preparative separation conditions: column: XBridge PrepOBD C18, 30*150mm, 5μm; column temperature: 25°C; mobile phase A: water (10mmol/L, $NH_4HCO_3$), mobile phase B: acetonitrile; flow rate: 60mL/min; the elution gradient is 25%B~70%B from 0 to 10 min, and 85%B after 10 min; detection wavelength: UV220nm; retention time (min): 6.8.

$^1$H NMR (400 MHz, DMSO-$d_6$) 8.97 (s, 1H), 8.76 (d, *J* = 7.2 Hz, 1H), 8.52 (s, 1H), 7.97 (dd, *J* = 9.2, 1.6 Hz, 1H), 7.68 - 7.59 (m, 3H), 7.31 (t, *J* = 74.4 Hz, 1H), 7.22 - 7.16 (m, 2H), 7.06 (d, *J* = 7.2 Hz, 1H), 4.20 (s, 3H), 2.19 (dq, *J* = 8.1, 4.6, 4.0 Hz, 1H), 1.13 (dd, *J* = 7.8, 3.5 Hz, 2H), 1.02 (t, *J* = 3.8 Hz, 2H).
LCMS m/z= 460[M+1]$^+$

**Test Example 1: Bioactivity Activity testing**

**I. MAT2A enzymatic testing method**

**1. Experimental steps**

a) 5×MAT2A assay-buffer (250mM Tris-HCl, pH8.0; 250mM KCl; 75mM $MgCl_2$; 0.025% BSA; 0.05%Brij35; 1.5mM EDTA) were first prepared and partially diluted to 1× for further use;
b) Preparation and addition of MAT2A enzyme (BPS, 71401): MAT2A enzyme was prepared to 3.674ng/μL (1.67×, final concentration 2.20ng/μL) with 1× MAT2A assay-buffer. With BioTek (MultiFlo FX) automatic dispenser, 15μL 1.67 ×MAT2A enzyme solution were added to the compound test well and negative control well respectively. At the same time, 15μL of 1×MAT2A assay-buffer was added to the blank control well;

c) Preparation and addition of compounds: the compound to be tested was diluted from 10mM stock solution to 100μM with DMSO. The positive drug AGI-24512 was diluted under the same conditions. Tecan compound titrator (D300e) was applied to automatically spray the solution into each well according to the preset concentration gradient, with the dispensed volume being extremely small and negligible. The starting concentration gradient is 1μM, 1/2 log, and a total of 8 gradients are set. The plate was centrifuged at 2500rpm for 30s and incubated at 25°C for 30min;
d) Preparation of ATP: 10mM ATP (Sigma, A7699) was diluted to 700μM with 1×MAT2A assay-buffer for further use;
e) Preparation and addition of substrate and ATP mixture: 5×MAT2A assay-buffer, 3μL/well; 750μM L-methionine (Adamas, 01100469), 2.5μL/well; 700μM ATP, 2.5μL/well; double distilled water, 2μL/well. With total amount of mixed solution required determined according to the number of detection wells, using BioTek (MultiFlo FX) automatic dispenser, 10 μL of the mixed solution was added to each well; The plate was centrifuged at 2500rpm for 30s and reacted at 25°C for 150min;
f) Addition of Biomol Green detection reagent: with BioTek (MultiFlo FX) automatic dispenser, 50μL Biomol Green (Enzo, BML-AK111) was added to each well, the plate was centrifuged at 2500rpm for 30s and incubated at 25°C for 20min;
g) Upon completion of the reaction, the $OD_{620}$ value was obtained using Perkin Elmer (Envision 2105) multifunctional plate reader.

**2. Data analysis**

The inhibition rate was calculated through following equation:

$$\text{Inhibition rate\%} = \frac{\text{ODmax} - \text{ODsample}}{\text{ODmax} - \text{ODmin}} \times 100\%$$

wherein

OD sample: $OD_{620}$ value of the sample well;
ODmin: represents the average $OD_{620}$ value of blank control wells without enzyme and test compound;
ODmax: represents the average $OD_{620}$ value of negative control wells with enzyme but no tested compound.

Then GraphPad Prism 5 software is used to fit a dose-effect curve: log(inhibitor) vs. response -Variable slope, the $IC_{50}$ value of the compound's inhibition of MAT2A enzyme was obtained.

**II. Cell testing method**

**1. Experimental steps**

HCT116 MTAP-/-cells (purchased from Horizon Discovery): human colorectal cancer cell line with MTAP gene deletion was cultured using medium RPMI 1640 + 10% FBS (Fetal bovine serum). On day 0 of the experiment, the viable cell density of the above cells in the logarithmic growth phase was adjusted to 5000 cells/ml. The cell suspension was plated into a 96-well plate by 100 μl/well, and a blank group was set up in parallel; the inoculated cell plate was placed in an incubator at 37°C and 5% $CO_2$ and cultured overnight.

On day 1 of the experiment, the cell plate cultured overnight was removed, the supernatant was discarded, with 80 μl of serum-free RPMI 1640 medium added to each well, the plate was placed it in an incubator for starvation cultivation for 4 h. The test compound was dissolved in DMSO (Dimethyl sulfoxide) to prepare and obtain a 10 mM compound stock solution. After the starvation was completed, the cell plate was taken out, with 80 μl RPMI 1640+20% FBS medium added to each well, placed on the automatic liquid dosing instrument D300e (Tecan). The dosing program was set as follows: the highest concentration of the compound tested is 30 μM, DMSO is used for 3-fold concentration gradient dilution, a total of 10 concentrations, two duplicate wells are set for each concentration. The final concentration of DMSO for each well of the 96-well plate is 0.3%, v/v. The pre-prepared 10 mM stock solution of the compound to be tested went through the above dosing procedure to add the drug. After the dosing is completed, the cell plate was placed in the incubator and cultured for 120 h.

On day 6 of the experiment, the cell plate was taken out, with 50 μl CellTiter-Glo® (purchased from Promega) added to each well, the fluorescence signal was measured on Envision (PerkinElmer) according to the instruction manual.

**2. Data analysis**

GraphPad Prism 5 software is used to fit a dose-effect curve: log(inhibitor) vs. response - Variable slope, the $IC_{50}$ value of the compound for inhibiting cell proliferation was obtained. The inhibition rate was calculated through following equation:

$$\text{Inhibition rate } \% = \frac{1-(\text{Test substance signal value}-\text{Blank group signal value})}{\text{Negative control group signal value}-\text{Blank group signal value}} \times 100\ \%$$

wherein

Test substance signal value: averaged fluorescence signal value of cells + culture medium + compound group; Blank group signal value: averaged fluorescence signal value of culture medium group; Negative control group signal value: averaged fluorescence signal value of cells + culture medium group.

**III. Experimental results:**

According to the above experimental method, the $IC_{50}$ of AGI-24512 inhibiting MAT2A is 26.79nM. Chemical formula of AGI-24512:

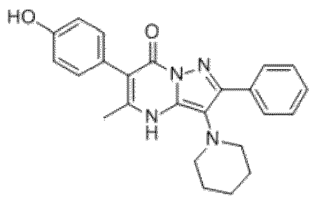

.

The experimental results of the compounds of the present disclosure are shown in Table 1 below:

Table 1

| Example number | MAT2A $IC_{50}$ (nM) | HCT116 MTAP-/- $IC_{50}$ (nM) |
|---|---|---|
| 1 | 6.44 | 259.8 |
| 2 | 11.36 | 118.6 |
| 3 | 10.73 | 14.33 |

**Test Example 2: In vivo pharmacokinetic studies in ICR mice**

**1. Experimental Procedure**

Male ICR mice (6 to 10 weeks old, Beijing Vital River Laboratory Animal Technology Co., Ltd.) were kept in SPF animal rooms at a temperature of 20 to 25°C, a relative humidity of 40% to 70%, and light and dark lighting for 12 hours each; animals had free access to water and food. After normal feeding for at least 5 days, mice with good physical condition after veterinary examination can be selected for this experiment. Each mouse is marked with a number on its tail.

The day before the experiment, the mice were fasted overnight and had free access to water. They were fed 4 hours after administration. The compound was prepared into a 20 mg/ml stock solution with DMSO, and the 20 mg/ml stock solution was accurately pipetted into a glass bottle with appropriate volume. An appropriate volume of PEG400 was added, mixed and then propylene glycol (PG) was added. The proportion of solvent in the final preparation is DMSO: PEG400:PG(v/v/v) = 5:65:30. The administration test solution of each compound to be tested with a concentration of 1 mg/ml was obtained.

The mice were weighted, the theoretical dosage volume of each mouse was calculated according to the following equation. The actual dosage of each mouse and the collection time of blood samples needed to be recorded in detail in the corresponding table.

$$\text{Theoretical dosage volume (mL)} = \left(\frac{\text{Dosage}(\text{mg} \cdot \text{kg}^{-1})}{\text{Test concentration } (\text{mg} \cdot \text{mL}^{-1})}\right) \times \text{Animal body weight(kg)}$$

On the day of the experiment, mice in each group received intragastric administration of a test solution of each compound to be tested at a dose of 10 mg/kg. After administration, approximately 40 μL of blood was collected from the orbit of the mice at each time point and placed in an EDTA-K2 anticoagulant tubes. The whole blood sample was centrifuged at 1500~1600g for 10 min, and the separated plasma was stored in a -40~-20°C refrigerator for biological sample analysis.

**2. Data analysis**

The LC-MS/MS analysis method was used to determine the concentration of the compounds in the biological samples obtained from the experiment. The non-compartmental model in Pharsight Phoenix 7.0 was used to calculate the pharmacokinetic parameters.

**3. Experimental results**

The calculations of pharmacokinetic parameters are shown in Table 2 below.

Table 2

| Example | Parameters | | |
|---|---|---|---|
| 1 | PO @ 10 mg/kg | Peak Concentration Cmax ($ng \cdot mL^{-1}$) | 2070 |
| | | Area Under Curve $AUC_{0-t}$ ($ng \cdot h \cdot mL^{-1}$) | 45500 |
| 2 | PO @ 10 mg/kg | Peak Concentration Cmax ($ng \cdot mL^{-1}$) | 1370 |
| | | Area Under Curve $AUC_{0-t}$ ($ng \cdot h \cdot mL^{-1}$) | 8730 |
| 3 | PO @ 10 mg/kg | Peak Concentration Cmax ($ng \cdot mL^{-1}$) | 4210 |
| | | Area Under Curve $AUC_{0-t}$ ($ng \cdot h \cdot mL^{-1}$) | 78300 |

**Test Example 3: HCT116 $MTAP^{-/-}$ In vivo drug efficacy experiment on subcutaneous xenograft tumors in nude mice**

**1. Experimental Procedure**

Female Nu/Nu nude mice (6-8 weeks old, Beijing Vital river Experimental Animal Technology Co., Ltd.) were kept in an SPF animal room with a temperature of 20~25°C, a relative humidity of 40%~70%, and light and dark lighting for 12 hours each; The animals had free access to water and food. The animals were adaptively reared before the experiment.

HCT116 $MTAP^{-/-}$ cells (Horizon) were cultured and amplification in vitro. The cells in the logarithmic growth phase were harvested and resuspended in serum-free RPMI-1640 medium. The cell concentration was adjusted to $6.0 \times 10^7$ cells/mL; the cell suspension was injected subcutaneously into the front right axilla of nude mice with a 1mL syringe for 100 μL into each animal. The animal condition was observed regularly for monitoring the growth of the transplanted tumors.

When tumor volume reaches 100~300 $mm^3$, animals with tumors that are too large, too small or whose tumor formation is uncertain was eliminated. Tumor-bearing mice with good health and similar tumor volumes was selected and divided into random groups; the treatment group received daily intragastric administration (AG-270: 50 mg/kg, chemical formula as shown below; compound to be tested: 5 mg/kg), and the control group received intragastric administration the same volume of blank solvent daily. During the administration period, tumor dimensions were measured twice a week to calculate the tumor volume. Simultaneously, the animal body weight was measured and recorded. Detection of SAM in transplanted tumors: at the end of the experiment, the animals were euthanized by $CO_2$, the tumor tissues were removed, washed with cold PBS, weighed, quick-frozen in liquid nitrogen and stored at low temperature (-80°C) for further use. The frozen tumor tissues were taken out, after thawing in an ice bath, added to 80% methanol aqueous solution (containing 1 M formic acid). The ratio of tumor tissue to methanol aqueous solution (containing 1 M

formic acid) is 1: 10 (w/v), and tissue homogenization was performed; after homogenization, the homogenate was collected. After treating, SAM (S-adenosylmethionine) was detected with LC-MS/MS.
Chemical formula of AG-270:

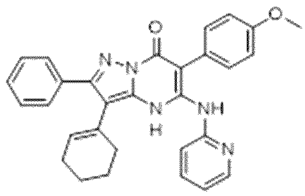

.

**2. Data analysis**

The equation to calculate tumor volume (TV) is:

$$TV=1/2\times a\times b^2;$$

wherein a represents the long diameter of the tumor, and b represents the short diameter of the tumor.
The equation to calculate relative tumor volume (RTV) is:

$$RTV = TV_t/TV_{initial};$$

wherein $TV_{initial}$ is the tumor volume initially measured at time of grouping and administration, and $TV_t$ is the tumor volume measured at each time during administration. The equation to calculate relative tumor proliferation rate (T/C (%)) is:

$$T/C\% = (RTV_T/RTV_C)\times 100\%;$$

wherein $RTV_T$ represents the relative tumor volume of the treatment group, RTVc represents the relative tumor volume of the solvent control group.
The equation to calculate tumor growth inhibition (TGI (%) ) is:

$$TGI=[1-(TV_{t(T)}-TV_{initial(T)})/(TV_{t(C)}-TV_{initial(C)})]\times 100\%;$$

wherein $TV_{t(T)}$ represents the tumor volume of the treatment group measured at each time, $TV_{initial(T)}$ represents the tumor volume of the treatment group initially measured at time of grouping and administration, $TV_{t(C)}$ represents the tumor volume of the solvent control group measured at each time, $TV_{initial(C)}$ represents the tumor volume of the solvent control group initially measured at time of grouping and administration.
The equation to calculate animal weight loss rate is:

$$\text{animal weight loss rate} = 100\% \times (BW_{initial}-BW_t)/BW_{initial};$$

wherein $BW_t$ represents the animal body weight measured at each time during administration, $BW_{initial}$ represents the animal weight initially measured at time of grouping and administration.
The equation to calculate tumor weight inhibition rate IR (%) is:

$$IR = 100\%\times(W_C-W_T)/W_C;$$

wherein Wc represents the tumor weight of the control group, $W_T$ represents the tumor weight of the treatment group.
Experimental data were calculated and statistically treated using Microsoft Office Excel 2007. Unless otherwise specified, data are expressed as mean ± standard error (Mean ± SE), and t-test was used for comparison between

two groups.

**3. Experimental results**

The tumor inhibition curve and intratumoral SAM inhibition results of HCT116 $MTAP^{-/-}$transplanted tumor model are shown in Figure 1 and Figure 2 respectively.

**Test Example 4: KP-4 In vivo drug efficacy experiment on subcutaneous xenograft tumors in mice**

**1. Experimental Procedures**

Female NOD SCID mice (6-8 weeks old, Beijing Vitalriver Experimental Animal Technology Co., Ltd.) were kept in an SPF animal room with a temperature of 20~25°C, a relative humidity of 40%~70%, and light and dark lighting for 12 hours each; The animals had free access to water and food. The animals were adaptively reared before the experiment.

KP-4 cells (Nanjing Kebai Biotechnology Co., Ltd.) were cultured and amplification in vitro. The cells in the logarithmic growth phase were harvested and resuspended in serum-free RPMI-1640 medium. The cell concentration was adjusted to $1.0\times10^8$ cells/mL; the cell suspension was injected subcutaneously into the front right axilla of nude mice with a 1mL syringe for 100 μL into each animal. The animal conditions was observed regularly for monitoring the growth of the transplanted tumors.

When tumor volume reaches 80~100 $mm^3$, animals with tumors that are too large, too small or whose tumor formation is uncertain was eliminated. Tumor-bearing mice with good health and similar tumor volumes was selected and divided into random groups; the treatment group received daily intragastric administration (AG-270: 100 mg/kg, compound to be tested: 5 mg/kg), and the control group received intragastric administration the same volume of blank solvent daily. During the administration period, tumor dimensions were measured twice a week to calculate the tumor volume. Simultaneously, the animal body weight was measured and recorded.

Detection of SAM in transplanted tumors: at the end of the experiment, the animals were euthanized by $CO_2$, the tumor tissues were removed, washed with cold PBS, weighed, quick-frozen in liquid nitrogen and stored at low temperature (-80°C) for further use. The frozen tumor tissues were taken out, after thawing in an ice bath, added to 80% methanol aqueous solution (containing 1 M formic acid). The ratio of tumor tissue to methanol aqueous solution (containing 1 M formic acid) is 1: 10 (w/v), and tissue homogenization was performed; after homogenization, the homogenate was collected. After treating, SAM (S-adenosylmethionine) was detected with LC-MS/MS.

**2. Data analysis**

The equation to calculate tumor volume (TV) is:

$$TV=1/2\times a\times b^2;$$

wherein a represents the long diameter of the tumor, and b represents the short diameter of the tumor.

The equation to calculate relative tumor volume (RTV) is:

$$RTV = TV_t/TV_{initial};$$

wherein $TV_{initial}$ is the tumor volume initially measured at time of grouping and administration, and $TV_t$ is the tumor volume measured at each time during administration. The equation to calculate relative tumor proliferation rate (T/C (%)) is:

$$T/C\% = (RTV_T/RTV_C)\times100\%;$$

wherein $RTV_T$ represents the relative tumor volume of the treatment group, RTVc represents the relative tumor volume of the solvent control group.

The equation to calculate tumor growth inhibition (TGI (%)) is:

$$TGI=[1-(TV_{t(T)}-TV_{initial(T)})/(TV_{t(C)}-TV_{initial(C)})]\times 100\%;$$

wherein $TV_{t(T)}$ represents the tumor volume of the treatment group measured at each time, $TV_{initial(T)}$ represents the tumor volume of the treatment group initially measured at time of grouping and administration, $TV_{t(C)}$ represents the tumor volume of the solvent control group measured at each time, $TV_{initial(C)}$ represents the tumor volume of the solvent control group initially measured at time of grouping and administration.

The equation to calculate animal weight loss rate is:

$$\text{animal weight loss rate} = 100\% \times (BW_{initial}-BW_t)/BW_{initial};$$

wherein $BW_t$ represents the animal body weight measured at each time during administration, $BW_{initial}$ represents the animal weight initially measured at time of grouping and administration.

The equation to calculate tumor weight inhibition rate IR (%) is:

$$IR = 100\%\times(W_C-W_T)/W_C;$$

wherein Wc represents the tumor weight of the control group, $W_T$ represents the tumor weight of the treatment group.

Experimental data were calculated and statistically treated using Microsoft Office Excel 2007. Unless otherwise specified, data are expressed as mean ± standard error (Mean ± SE), and t-test was used for comparison between two groups.

### 3. Experimental results

The tumor inhibition curve and intratumorally SAM inhibition results of KP-4 transplanted tumor model are shown in Figure 3 and Figure 4 respectively.

## Claims

**1.** A compound of formula I or a pharmaceutically acceptable salt thereof:

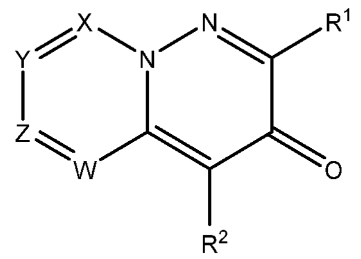

I

wherein X is selected from $CR^3$ or N; Y is selected from $CR^4$ or N; Z is selected from $CR^5$ or N; W is selected from $CR^6$ or N;

wherein $R^3$, $R^4$, $R^5$ and $R^6$ are independently selected from hydrogen, cyano, C2-C6 alkynyl, halogen, hydroxyl, $-NH_2$, (C1-C6 alkyl)-$NR^7$-, (C1-C6 alkyl)-O-, (C1-C6 alkyl)-S-, C1-C6 alkyl, C3-C6 cycloalkyl, 6- to 10-membered aryl, C2-C6 alkenyl or C3-C6 cycloalkenyl; the C1-C6 alkyl, C2-C6 alkenyl, C3-C6 cycloalkyl or C3-C6 cycloalkenyl itself or as part of another group is optionally substituted by halogen, cyano, hydroxyl, $-NR^7R^8$, C1-C3 alkyl, C1-C3 alkoxy, C2-C6 alkenyl or C2-C6 alkynyl, the 6- to 10-membered aryl is optionally substituted by halogen, hydroxyl, cyano, $-NR^7R^8$, $-NO_2$, C1-C3 alkyl, C1-C3 alkoxy, C2-C6 alkenyl or C2-C6 alkynyl, wherein the C1-C3 alkyl, C1-C3 alkoxy, C2-C6 alkenyl or C2-C6 alkynyl is optionally substituted by halogen, hydroxyl, cyano, $-NR^7R^8$ or $-NO_2$;

$R^1$ and $R^2$ are independently selected from 6- to 10-membered aryl or 9- to 18-membered benzoheterocyclyl, the 6- to 10-membered aryl or 9- to 18-membered benzoheterocyclyl is optionally substituted by halogen, hydroxyl, cyano, $-NR^7R^8$, $-NO_2$, $-NR^9C(O)R^{10}$, C1-C6 alkyl, (C1-C6 alkyl)-O-, $-C(O)NR^9R^{10}$ or 5- to 7-membered

heteroaryl, the C1-C6 alkyl itself or as part of another group or the 5- to 7-membered heteroaryl is optionally substituted by halogen, cyano, hydroxyl, C1-C3 alkyl, (C1-C3 alkyl)-O- or $-NR^7R^8$;
$R^7$, $R^8$, $R^9$ and $R^{10}$ are independently selected from H or C1-C6 alkyl;
provided that: at most 2 of W, X, Y and Z are simultaneously N.

**2.** The compound of formula I according to claim 1, or a pharmaceutically acceptable salt thereof, wherein the compound of formula I having the structure of formula II,

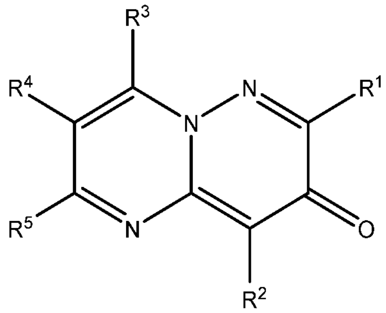

II ,

wherein $R^1$, $R^2$, $R^3$, $R^4$ and $R^5$ are defined as the compound of formula I.

**3.** The compound according to claim 1 or 2, or a pharmaceutically acceptable salt thereof, wherein $R^3$, $R^4$, $R^5$ and $R^6$ are independently selected from hydrogen, halogen, hydroxyl, - $NH_2$, (C1-C6 alkyl)-$NR^7$-, (C1-C6 alkyl)-O-, C1-C6 alkyl, C3-C6 cycloalkyl or 6- to 10-membered aryl, wherein the C1-C6 alkyl itself or as part of another group or the C3-C6 cycloalkyl is optionally substituted by halogen, cyano, hydroxyl or $-NR^7R^8$, the 6- to 10-membered aryl is optionally substituted by halogen-substituted C1-C3 alkoxy;

preferably, $R^3$, $R^4$, $R^5$ and $R^6$ are independently selected from hydrogen, halogen, hydroxyl, $-NH_2$, (C1-C6 alkyl)-$NR^7$-, (C1-C6 alkyl)-O-, C1-C6 alkyl, C3-C6 cycloalkyl or 6- to 10-membered aryl, wherein the C1-C6 alkyl itself or as part of another group or the C3-C6 cycloalkyl is optionally substituted by halogen, the 6- to 10-membered aryl is optionally substituted by halogen-substituted C1-C3 alkoxy;
more preferably, $R^3$, $R^4$, $R^5$ and $R^6$ are independently selected from hydrogen, halogen, hydroxyl, $-NH_2$, (C1-C6 alkyl)-$NR^7$-, (C1-C6 alkyl)-O-, C1-C6 alkyl, C3-C6 cycloalkyl or 6- to 10-membered aryl, wherein the C1-C6 alkyl itself or as part of another group or C3-C6 cycloalkyl is optionally substituted by fluorine, the 6- to 10-membered aryl is optionally substituted by fluorine-substituted C1-C3 alkoxy.

**4.** The compound according to any one of claims 1-3, or a pharmaceutically acceptable salt thereof, wherein $R^3$ is selected from hydrogen, C1-C6 alkyl or C3-C6 cycloalkyl; preferably, $R^3$ is selected from hydrogen or C1-C6 alkyl; more preferably, $R^3$ is selected from hydrogen, methyl or cyclopropyl; more preferably, $R^3$ is selected from hydrogen or methyl; most preferably, $R^3$ is hydrogen.

**5.** The compound according to any one of claims 1-4, or a pharmaceutically acceptable salt thereof, wherein $R^4$ is selected from hydrogen or C1-C6 alkyl; preferably, $R^4$ is selected from hydrogen or methyl; more preferably, $R^4$ is hydrogen.

**6.** The compound according to any one of claims 1-5, or a pharmaceutically acceptable salt thereof, wherein $R^5$ is selected from hydrogen, halogen, hydroxyl, (C1-C6 alkyl)-$NR^7$-, (C1-C6 alkyl)-O-, C3-C6 cycloalkyl or 6- to 10-membered aryl, wherein the C1-C6 alkyl itself or as part of another group or the C3-C6 cycloalkyl is optionally substituted by fluorine, the 6- to 10-membered aryl is optionally substituted by fluorine-substituted C1-C3 alkoxy; preferably, $R^5$ is selected from hydrogen, chlorine, hydroxyl, cyclopropyl, $CF_3CH_2O$-, $CHF_2O$-, $CF_3CH_2NH$-, 4-difluoromethoxyphenyl or $CH_3CH_2O$-; more preferably, $R^5$ is selected from cyclopropyl, $CF_3CH_2O$-, $CF_3CH_2NH$- or $CH_3CH_2O$-; more preferably, $R^5$ is selected from cyclopropyl, $CF_3CH_2O$- or $CF_3CH_2NH$-; most preferably, $R^5$ is cyclopropyl.

**7.** The compound according to any one of claims 1-6, or a pharmaceutically acceptable salt thereof, wherein $R^1$ and $R^2$ are independently selected from phenyl,

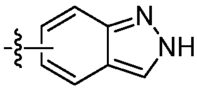

or

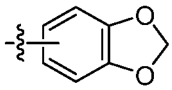

group, wherein the groups are optionally substituted by halogen, hydroxyl, cyano, $-NR^7R^8$, $-NO_2$, $-NR^9C(O)R^{10}$, C1-C6 alkyl, (C1-C6 alkyl)-O-, $-C(O)NR^9R^{10}$ or 5- to 7-membered heteroaryl, the C1-C6 alkyl itself or as part of another group or the 5- to 7-membered heteroaryl is optionally substituted by halogen, cyano, hydroxyl, C1-C3 alkyl, (C1-C3 alkyl) -O- or $-NR^7R^8$; preferably, $R^1$ and $R^2$ are independently selected from phenyl,

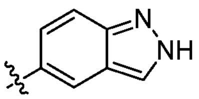 , 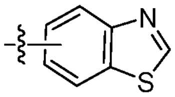 , 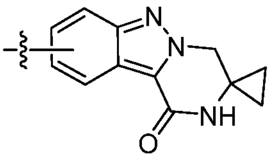 , 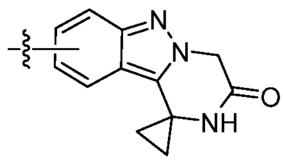 ,

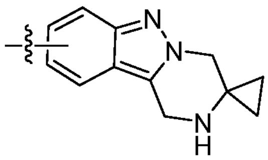 , 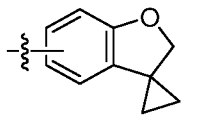 or 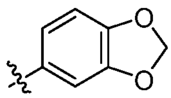

group, wherein the groups are optionally substituted by C1-C6 alkyl or (C1-C6 alkyl)-O-, the C1-C6 alkyl itself or as part of another group is optionally substituted by halogen; preferably, $R^7$, $R^8$, $R^9$ and $R^{10}$ are independently selected from H.

**8.** The compound according to any one of claims 1-7, or a pharmaceutically acceptable salt thereof, wherein $R^1$ is selected from phenyl,

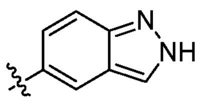 or 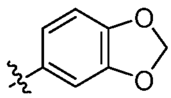

group, wherein the groups are optionally substituted by C1-C6 alkyl or (C1-C6 alkyl)-O-, the C1-C6 alkyl itself or as part of another group is optionally substituted by halogen; preferably, $R^1$ is selected from

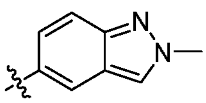 , 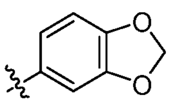 or 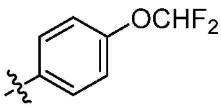 ;

more preferably, $R^1$ is

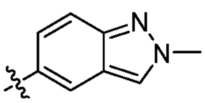 .

**9.** The compound according to any one of claims 1-8, or a pharmaceutically acceptable salt thereof, wherein $R^2$ is selected from phenyl or

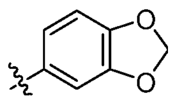

group, wherein the groups are optionally substituted by (C1-C6 alkyl)-O-, the C1-C6 alkyl is optionally substituted by halogen; preferably, $R^2$ is selected from

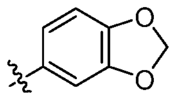 or 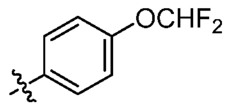 ;

more preferably, $R^2$ is

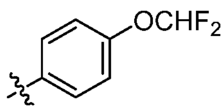 .

**10.** A compound selected from the following group, or a pharmaceutically acceptable salts thereof:

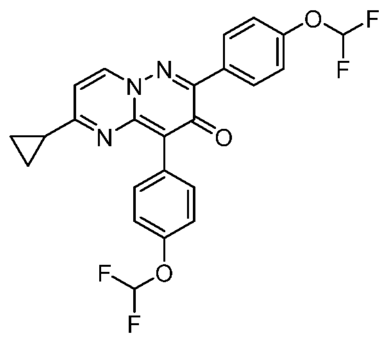 ,

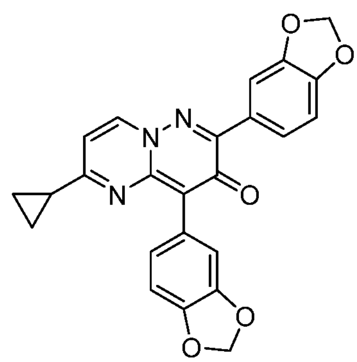

and

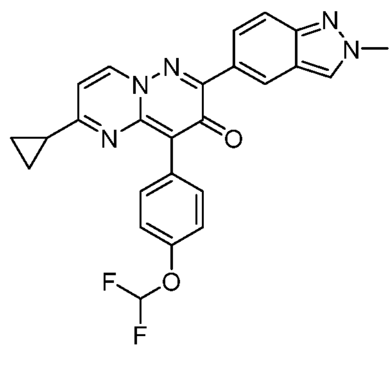 .

**11.** A pharmaceutical composition, comprising a therapeutically effective amount of a compound of any one of claims 1 to 10 or a pharmaceutically acceptable salt thereof.

**12.** Use of a compound of any one of claims 1 to 10, a pharmaceutically acceptable salt thereof or a pharmaceutical composition of claim 11 in the preparation of a medicament for preventing and/or treating a MAT2A mediated disease or condition; preferably, the MAT2A mediated disease or condition is mediated by overexpression of MAT2A.

**13.** A method for preventing and/or treating a MAT2A mediated disease or condition, comprising administering to a

subject in need an effective amount of a compound of any one of claims 1 to 10, a pharmaceutically acceptable salt thereof, or a pharmaceutical composition of claim 11.

**14.** The compound of any one of claims 1 to 10, a pharmaceutically acceptable salt thereof, or the pharmaceutical composition of claim 11 for use in the prevention and/or treatment of a MAT2A mediated disease or condition.

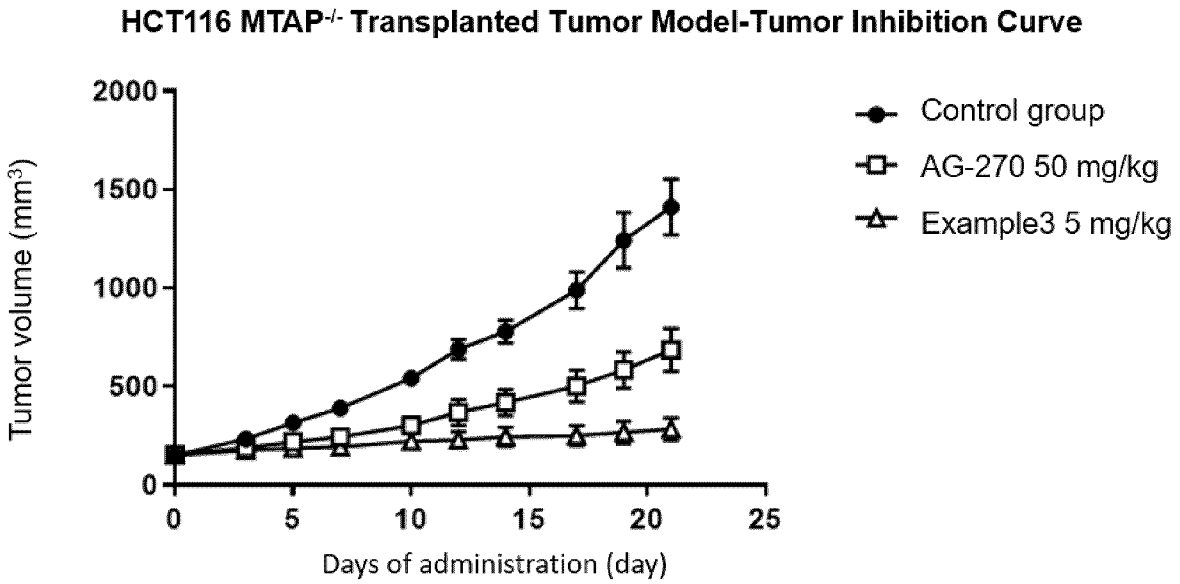
HCT116 MTAP-/- Transplanted Tumor Model-Tumor Inhibition Curve
Tumor volume (mm3)
2000
1500
1000
500
0
0
5
10
15
20
25
Days of administration (day)
Control group
AG-270 50 mg/kg
Example3 5 mg/kg

FIG. 1

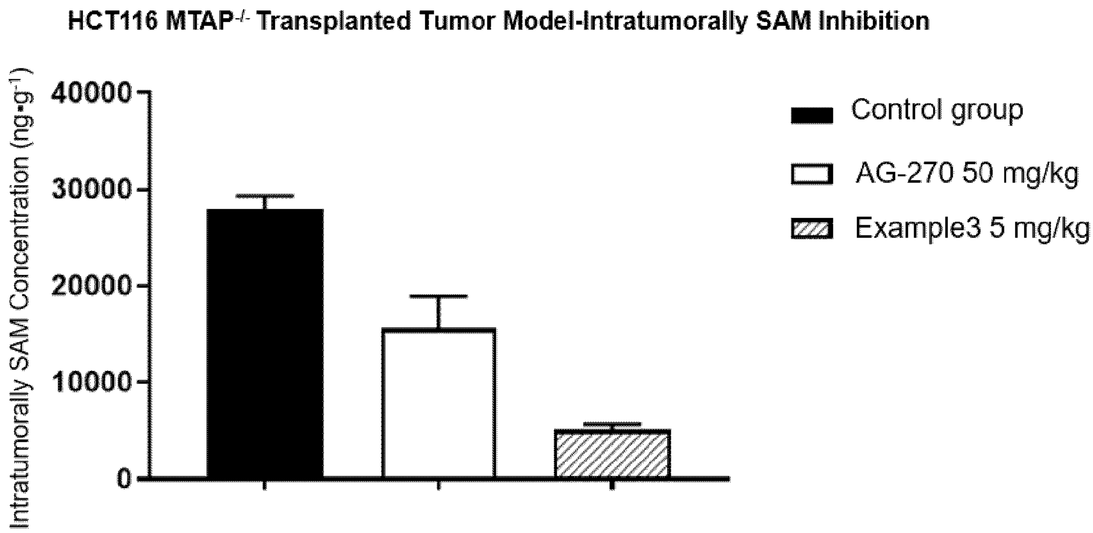
HCT116 MTAP-/- Transplanted Tumor Model-Intratumorally SAM Inhibition
Intratumorally SAM Concentration (ng•g-1)
40000
30000
20000
10000
0
Control group
AG-270 50 mg/kg
Example3 5 mg/kg

FIG. 2

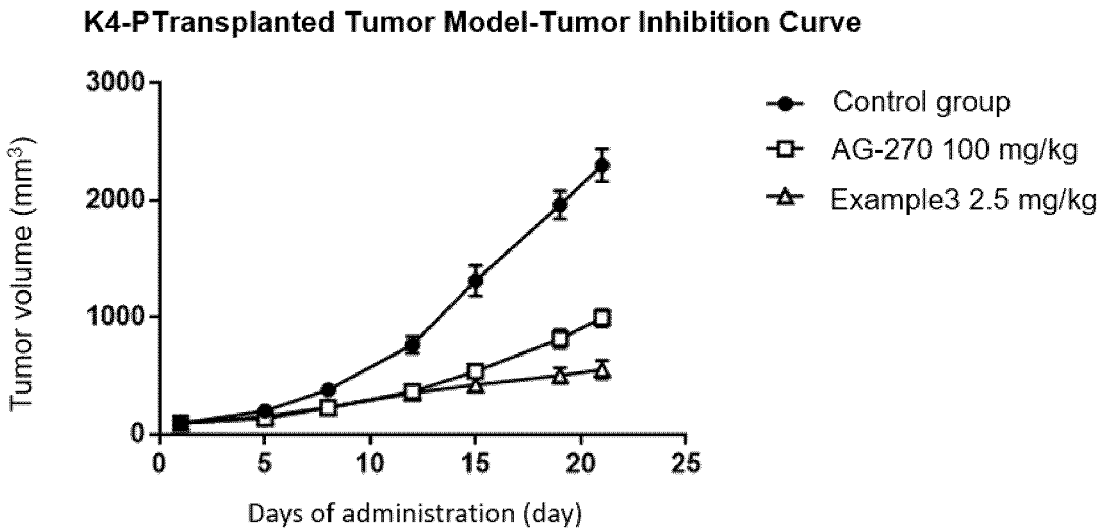
K4-PTransplanted Tumor Model-Tumor Inhibition Curve
3000
2000
1000
0
Tumor volume (mm3)
0
5
10
15
20
25
Days of administration (day)
Control group
AG-270 100 mg/kg
Example3 2.5 mg/kg

FIG. 3

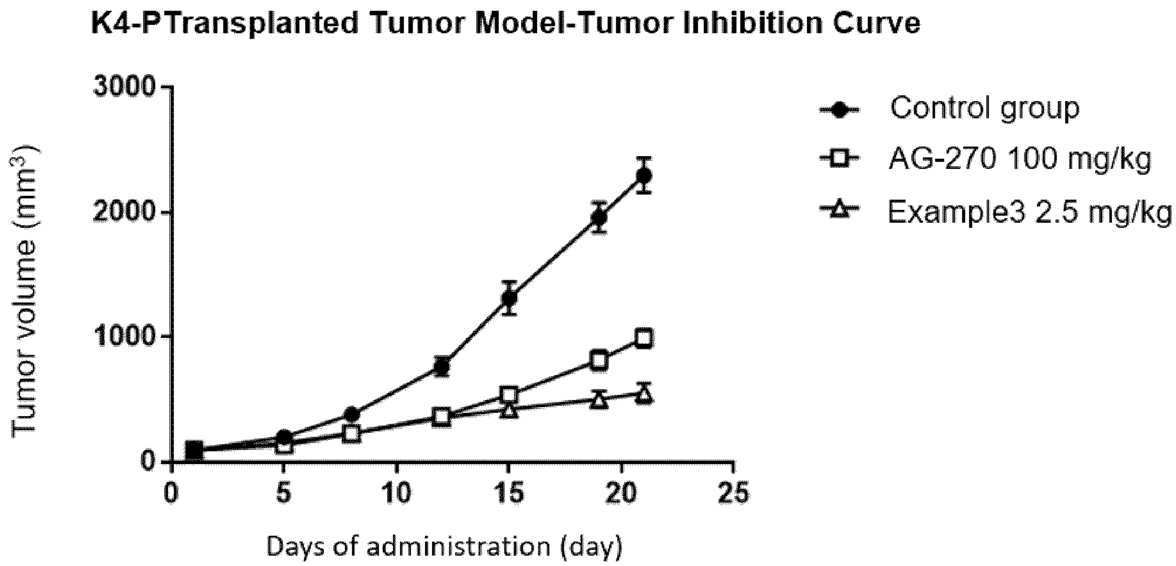
K4-PTransplanted Tumor Model-Tumor Inhibition Curve
3000
2000
1000
0
Tumor volume (mm3)
0
5
10
15
20
25
Days of administration (day)
Control group
AG-270 100 mg/kg
Example3 2.5 mg/kg

FIG. 4

## INTERNATIONAL SEARCH REPORT

International application No. **PCT/CN2022/140380**

**A. CLASSIFICATION OF SUBJECT MATTER**

C07D487/04(2006.01)i;C07D471/20(2006.01)i;C07D471/04(2006.01)i;A61K31/5025(2006.01)i;A61P35/00(2006.01)i

According to International Patent Classification (IPC) or to both national classification and IPC

**B. FIELDS SEARCHED**

Minimum documentation searched (classification system followed by classification symbols)

C07D, A61K, A61P

Documentation searched other than minimum documentation to the extent that such documents are included in the fields searched

Electronic data base consulted during the international search (name of data base and, where practicable, search terms used)

CNABS, CNTXT, DWPI, WPABS, CAPLUS(STN), REGISTRY(STN), MARPAT(STN), CNKI: 结构式检索, search for structural formula, MAT2A, MAT, MTAP, 甲硫氨酸腺苷转移酶, 甲硫腺苷磷酸化酶, 嘧啶, 哒嗪酮, pyridine, pyridezinone

**C. DOCUMENTS CONSIDERED TO BE RELEVANT**

| Category* | Citation of document, with indication, where appropriate, of the relevant passages | Relevant to claim No. |
|---|---|---|
| A | CN 111936499 A (AGIOS PHARMACEUTICALS, INC.) 13 November 2020 (2020-11-13) claims 1, 34-40, description, paragraphs 9-28, embodiment 277 | 1-14 |
| A | CN 113454085 A (LES LABORATOIRES SERVIER SAS) 28 September 2021 (2021-09-28) claims 1, 24, description, paragraphs 10-21 | 1-14 |
| A | CN 113474347 A (LES LABORATOIRES SERVIER SAS) 01 October 2021 (2021-10-01) claims 1, 35 | 1-14 |
| A | WO 2021139775 A1 (JIANGSU SIMCERE PHARMACEUTICAL CO., LTD.) 15 July 2021 (2021-07-15) claims 1, 12 | 1-14 |
| A | WO 2020243376 A1 (AGIOS PHARMACEUTICALS INC.) 03 December 2020 (2020-12-03) claims 1-59 | 1-14 |
| A | CN 113166078 A (IDEAYA BIOSCIENCES INC.) 23 July 2021 (2021-07-23) claims 1-54 | 1-14 |

☐ Further documents are listed in the continuation of Box C. ☑ See patent family annex.

* Special categories of cited documents:

"A" document defining the general state of the art which is not considered to be of particular relevance

"D" document cited by the applicant in the international application

"E" earlier application or patent but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **03 March 2023** | **10 March 2023** |
| Name and mailing address of the ISA/CN<br>**China National Intellectual Property Administration (ISA/CN)**<br>**China No. 6, Xitucheng Road, Jimenqiao, Haidian District, Beijing 100088**<br>Facsimile No. **(86-10)62019451** | Authorized officer<br><br>Telephone No. |

Form PCT/ISA/210 (second sheet) (July 2022)

**INTERNATIONAL SEARCH REPORT**

International application No.
**PCT/CN2022/140380**

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☑ Claims Nos.: **13**
because they relate to parts of the international application that do not comply with the prescribed requirements to such an extent that no meaningful international search can be carried out, specifically:

The subject matter of claim 13 belongs to the methods for treating diseases of a human body (PCT Rule 39.1(iv)). The search is carried out on the basis of the use of a compound and a salt or a composition thereof in the preparation of a drug for treating corresponding diseases.

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2022/140380**

| Patent document cited in search report | Publication date (day/month/year) | Patent family member(s) | Publication date (day/month/year) |
|---|---|---|---|
| CN 111936499 A | 13 November 2020 | MX 2020010005 A | 14 October 2020 |
| | | PH 12020551507 A1 | 13 September 2021 |
| | | TW 201946629 A | 16 December 2019 |
| | | TWI 719437 B | 21 February 2021 |
| | | US 2021115045 A1 | 22 April 2021 |
| | | US 11524960 B2 | 13 December 2022 |
| | | EP 3774805 A1 | 17 February 2021 |
| | | AR 115326 A1 | 23 December 2020 |
| | | EA 202092320 A1 | 09 March 2021 |
| | | MA 52232 A | 17 February 2021 |
| | | AU 2019243289 A1 | 24 September 2020 |
| | | AU 2019243289 B2 | 12 January 2023 |
| | | CA 3094476 A1 | 03 October 2019 |
| | | SG 11202009195 WA | 29 October 2020 |
| | | KR 20200138769 A | 10 December 2020 |
| | | BR 112020020104 A2 | 26 January 2021 |
| | | IL 277665 A | 30 November 2020 |
| | | JP 2021519783 A | 12 August 2021 |
| | | WO 2019191470 A1 | 03 October 2019 |
| | | WO 2019191470 A8 | 12 December 2019 |
| CN 113454085 A | 28 September 2021 | IL 284326 A | 31 August 2021 |
| | | CR 20210410 A | 08 December 2021 |
| | | AU 2019416349 A1 | 15 July 2021 |
| | | CA 3124952 A1 | 02 July 2020 |
| | | SG 11202106637 SA | 29 July 2021 |
| | | US 2022144820 A1 | 12 May 2022 |
| | | KR 20220051301 A | 26 April 2022 |
| | | TW 202039490 A | 01 November 2020 |
| | | WO 2020139991 A1 | 02 July 2020 |
| | | CO 2021009879 A2 | 29 October 2021 |
| | | AR 117544 A1 | 11 August 2021 |
| | | EA 202191801 A1 | 09 November 2021 |
| | | EP 3902803 A1 | 03 November 2021 |
| | | EP 3902803 B1 | 01 February 2023 |
| | | CL 2021001721 A1 | 18 February 2022 |
| | | PE 20212090 A1 | 04 November 2021 |
| | | BR 112021012595 A2 | 08 September 2021 |
| | | MA 54608 A | 06 April 2022 |
| | | JP 2022516883 A | 03 March 2022 |
| CN 113474347 A | 01 October 2021 | JP 2022516882 A | 03 March 2022 |
| | | EA 202191800 A1 | 13 September 2021 |
| | | CO 2021009882 A2 | 29 October 2021 |
| | | TW 202039489 A | 01 November 2020 |
| | | EP 3902804 A1 | 03 November 2021 |
| | | CL 2021001722 A1 | 18 February 2022 |
| | | MA 54609 A | 06 April 2022 |
| | | WO 2020139992 A1 | 02 July 2020 |
| | | AU 2019414446 A1 | 15 July 2021 |
| | | IL 284324 A | 31 August 2021 |
| | | KR 20220050832 A | 25 April 2022 |

Form PCT/ISA/210 (patent family annex) (July 2022)

**INTERNATIONAL SEARCH REPORT**
**Information on patent family members**

International application No.
**PCT/CN2022/140380**

| Patent document cited in search report | | | Publication date (day/month/year) | Patent family member(s) | | | Publication date (day/month/year) |
|---|---|---|---|---|---|---|---|
| | | | | US | 2022098203 | A1 | 31 March 2022 |
| | | | | AR | 115296 | A1 | 16 December 2020 |
| | | | | CR | 20210409 | A | 24 January 2022 |
| | | | | CA | 3124678 | A1 | 02 July 2020 |
| | | | | BR | 112021012599 | A2 | 08 September 2021 |
| | | | | PE | 20212303 | A1 | 10 December 2021 |
| | | | | SG | 11202106627 | WA | 29 July 2021 |
| WO | 2021139775 | A1 | 15 July 2021 | None | | | |
| WO | 2020243376 | A1 | 03 December 2020 | EP | 3976611 | A1 | 06 April 2022 |
| | | | | BR | 112021023825 | A2 | 08 February 2022 |
| | | | | SG | 11202112952 | SA | 30 December 2021 |
| | | | | CA | 3142340 | A1 | 03 December 2020 |
| | | | | AR | 119046 | A1 | 17 November 2021 |
| | | | | PE | 20220387 | A1 | 18 March 2022 |
| | | | | IL | 288395 | A | 01 January 2022 |
| | | | | CO | 2021017981 | A2 | 19 April 2022 |
| | | | | US | 2022251081 | A1 | 11 August 2022 |
| | | | | MA | 56050 | A | 06 April 2022 |
| | | | | TW | 202110841 | A | 16 March 2021 |
| | | | | JP | 2022534989 | A | 04 August 2022 |
| | | | | AU | 2020284018 | A1 | 27 January 2022 |
| | | | | CR | 20210670 | A | 11 February 2022 |
| CN | 113166078 | A | 23 July 2021 | IL | 283743 | A | 29 July 2021 |
| | | | | KR | 20210103498 | A | 23 August 2021 |
| | | | | CO | 2021008895 | A2 | 30 July 2021 |
| | | | | CL | 2021001508 | A1 | 21 January 2022 |
| | | | | US | 11130759 | B1 | 28 September 2021 |
| | | | | US | 11084798 | B1 | 10 August 2021 |
| | | | | JP | 2022512156 | A | 02 February 2022 |
| | | | | TW | 202039487 | A | 01 November 2020 |
| | | | | SG | 11202105469 | YA | 29 June 2021 |
| | | | | US | 2022106276 | A1 | 07 April 2022 |
| | | | | PH | 12021551274 | A1 | 06 December 2021 |
| | | | | MA | 54452 | A | 16 March 2022 |
| | | | | BR | 112021010842 | A2 | 24 August 2021 |
| | | | | EP | 3894396 | A1 | 20 October 2021 |
| | | | | US | 11046691 | B1 | 29 June 2021 |
| | | | | CA | 3121236 | A1 | 18 June 2020 |
| | | | | AU | 2019395338 | A1 | 29 July 2021 |
| | | | | WO | 2020123395 | A1 | 18 June 2020 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 109890822 A
- WO 2020123395 A1